(19) Europäisches Patentamt / European Patent Office / Office européen des brevets

(11) **EP 3 242 713 B1**

(12) **EUROPEAN PATENT SPECIFICATION**

(45) Date of publication and mention
of the grant of the patent:
**09.03.2022 Bulletin 2022/10**

(21) Application number: **16735397.8**

(22) Date of filing: **07.01.2016**

(51) International Patent Classification (IPC):
***A61N 1/372*** *(2006.01)*

(52) Cooperative Patent Classification (CPC):
**A61N 1/37252; A61N 1/37217; A61N 1/37254;**
A61N 1/37288

(86) International application number:
**PCT/US2016/012439**

(87) International publication number:
**WO 2016/112166 (14.07.2016 Gazette 2016/28)**

(54) **ULTRASONIC MULTIPLEXING NETWORK FOR IMPLANTABLE MEDICAL DEVICES**

ULTRASCHALLMULTIPLEXNETZWERK FÜR IMPLANTIERBARE MEDIZINISCHE
VORRICHTUNGEN

RÉSEAU DE MULTIPLEXAGE ULTRASONORE POUR DISPOSITIFS MÉDICAUX IMPLANTABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.01.2015 US 201562100628 P**

(43) Date of publication of application:
**15.11.2017 Bulletin 2017/46**

(73) Proprietor: **Northeastern University
Boston, MA 02115 (US)**

(72) Inventors:
• **MELODIA, Tommaso
Newton, MA 02459 (US)**
• **SANTAGATI, Giuseppe, Enrico
Cambridge, MA 02141 (US)**

(74) Representative: **Høiberg P/S
Adelgade 12
1304 Copenhagen K (DK)**

(56) References cited:
**EP-A1- 1 115 221        EP-A2- 1 924 027
US-A- 5 861 018         US-A1- 2002 141 479
US-A1- 2004 202 339     US-A1- 2007 238 482
US-A1- 2010 080 265     US-A1- 2011 077 715
US-A1- 2012 134 423     US-A1- 2013 033 966
US-A1- 2014 016 558     US-A1- 2014 269 396**

• **SANTAGATI ET AL.: 'Ultrasonic networking for
e-health applications.' IEEE WIRELESS
COMMUNICATIONS. August 2013, XP011526504
Retrieved from the Internet:
<URL:http://www.ece.neu.edu/winesiab/papers
/Santagati13WCM.pdf>**

**Description**

BACKGROUND

Implantable medical sensing and actuating devices with wireless capabilities are used in many digital health applications. Existing wireless medical implants are connected through radio frequency (RF) electromagnetic waves. RF-based solutions tend to scale down traditional wireless technologies, such as Wi-Fi or Bluetooth, to the intrabody environment, with little or no attention paid to the peculiar characteristics and safety requirements of the human body and to the privacy and security requirements of patients.

US 2013/033966 discloses an implantable system in which an intra-body ultrasonic signal is converted into an electrical signal, and where a local oscillator signal can be generated. The electric and local oscillator signal can be mixed in the implantable system, such as to generate a demodulated signal, processed, such as using a filter.

In telecommunications, orthogonal frequency-division multiplexing (OFDM) is a method of encoding digital data on multiple carrier frequencies. US 2014/016558 discloses an apparatus and method for transmitting data and power through a metal barrier by using ultrasonic waves based on OFDM. EP 1115221 discloses a communication system between a road and a vehicle which performs communication between a road communication station and a vehicle-mounted mobile station in a cell, utilizing an OFDM modulation method. US 2010/080265 discloses a communication device which communicate data and a training sequence corresponding to a preamble. A modulation and mapping circuit modulates the signals into a plurality of multiple subcarriers that are orthogonal to each other to form an OFDM communications signal. US 2014/269396 discloses a method and apparatus for allocating subcarriers in an orthogonal frequency division multiple access (OFDMA) system. The method may comprise allocating at least one diversity cluster of subcarriers to a first subscriber and allocating at least one coherence cluster to a second subscriber.

US 2007/238482 discloses devices, systems and methods of coordination among multiple transceivers. The document discloses a wireless transceiver able to operate in accordance with a non-scheduled wireless communication protocol and a second, scheduled, wireless transceiver able to operate in accordance with a second wireless communication protocol. US 2012/134423 discloses a method which includes receiving a stream of data bits, demultiplexing the stream into a first and second substream, encoding the first and second substream using a low density parity check coding process, and mapping the first substream to a first region of a symbol constellation map and the second substream to a second region.

SUMMARY OF THE INVENTION

The invention is defined in the appended claims. Aspects, embodiments and examples disclosed herein which do not fall within the scope of the appended claims do not form part of the invention, and are merely provided for illustrative purposes.

DESCRIPTION OF THE DRAWINGS

The invention will be more fully understood from the following detailed description taken in conjunction with the accompanying drawings in which:

Fig. 1 is a schematic diagram of an OFDM encoder (top) and decoder (bottom);
Fig. 2 is a schematic diagram of an example of pure time-hopping strategy (top), and a combined frequency- and time-hopping strategy (bottom); and
Fig. 3 is a schematic illustration of a network of nodes implanted in a body.

DETAILED DESCRIPTION OF THE INVENTION

Radio frequency (RF) technology presents several limitations that can negatively affect patients' medical experience and safety. First, RF waves do not propagate well in biological tissues, which leads to higher energy consumption and heating of the tissues. Second, the RF frequency spectrum is scarce, strictly regulated, and already crowded with many devices interfering with one another. Therefore, RF-based technologies raise serious concerns about potential interference from existing RF communication systems that can unintentionally undermine the reliability and security of an intra-body network, and ultimately the safety of the patient. Third, RF communications can be easily jammed, i.e., intentionally disrupted by artificially generated interference, or eavesdropped by malicious agents. This raises major privacy and security red flags for intra-body networks, and a risk for the patient. Fourth, the medical community is still divided on the risks caused by continuous exposure of human tissues to RF radiation. Therefore, a massive deployment of RF implantable devices may represent a potential risk for the patient. Finally, the dielectric nature of the human body also affects the coupling between on-body RF antennas and the body itself. In particular, the gain and the radiation pattern of the antenna deteriorate because of the contact or proximity with the human body, while the resonant frequency and the input impedance of the antenna may shift from their nominal values.

Accordingly, a communications system is described herein that uses ultrasonic waves as an alternative carrier of information in biological tissues. Ultrasonic waves are acoustic waves with frequency higher than the upper threshold for human hearing, i.e., generally 20 kHz. In some embodiments, the communications system can use near-ultrasonic waves, such as greater than 17 kHz. The ultrasonic communications system employs an orthogonal frequency division multiplexing (U-OFDM)-based networking scheme that offers link-to-link physical layer adaptation, with distributed control to enable multiple access among interfering implanted devices. U-OFDM is based on the idea of regulating the data rate of each transmitter to adapt to the current level of interference by distributively optimizing the physical layer parameter.

## 1. Ultrasonic Intra-Body Communications

Ultrasounds are mechanical waves that propagate in an elastic medium at frequencies above the upper limit for human hearing, i.e., 20 kHz.

**Attenuation.** Two main mechanisms contribute to ultrasound attenuation in tissues, i.e., absorption and scattering. An initial pressure $P_0$ decays at a distance d

$$P(d) = P_0 e^{-\alpha d}, \tag{1}$$

where $\alpha$ (in [Np $\cdot$ cm$^{-1}$]) is an amplitude attenuation coefficient that captures all the effects that cause dissipation of energy from the ultrasound wave. Parameter $\alpha$ depends on the carrier frequency through $\alpha = af^b$, where $f$ represents the carrier frequency (in MHz) and a (in [Np m$^{-1}$ MHz$^{-b}$]) and b are attenuation parameters characterizing the tissue.

**Propagation Speed.** Ultrasonic wave propagation is affected by propagation delays that are orders of magnitude higher than RF. The propagation speed of acoustic waves in biological tissues is approximately 1500m/s, as compared to $2 \times 10^8$ m/s for RF waves.

**Operating Frequency.** Considerations in determining the operating frequency are (i) the frequency dependence of the attenuation coefficient, and (ii) the frequency dependence of the beam spread of ultrasonic transducers (which is inversely proportional to the ratio of the diameter of the radiating surface and the wavelength). Therefore, higher frequencies help keep the transducer size small, but result in higher signal attenuation. Since most biomedical sensing applications require directional transducers, one needs to operate at the lowest possible frequencies compatible with small-size transducers and required signal bandwidth. For propagation distances in the order of several cm. the operating frequency should not exceed 10 MHz.

**Reflections and Scattering.** The human body is composed of different organs and tissues with different sizes, densities and sound speeds. Therefore, it can be modeled as an environment with pervasive presence of reflectors and scatterers. The direction and magnitude of the reflected wave depend on the orientation of the boundary surface and on the acoustic impedance of the tissues, while scattered reflections occur when an acoustic wave encounters an object that is relatively small with respect to its wavelength or a tissue with an irregular surface. Consequently, the received signal is obtained as the sum of numerous attenuated, possibly distorted, and delayed versions of the transmitted signal..

## 2. Ultrasonic Orthogonal Frequency Division Multiplexing (U-OFDM)

**OFDM.** Orthogonal frequency division multiplexing (OFDM) uses a large number of closely spaced orthogonal subcarriers, such that for each subcarrier the channel is subject to flat fading. In the time domain, this comprises dividing a high data rate stream into multiple low rate streams, each transmitted on a different subcarrier. In this way the symbol rate on each subcarrier is reduced, and hence the effect of intersymbol interference (ISI) caused by multipath delay spread is reduced. In each sub-carrier a conventional modulation scheme can be used, c.g., M-Phasc-Shift-Kcying (PSK) and M-Quadraturc-Amplitudc-Modulation (QAM). OFDM offers high spectral efficiency and robustness against narrow-band cochannel interference, intersymbol interference (ISI) and multipath fading. Assume a bandwidth $B$ divided in a set $F$ of subcarriers, $|F|$ being the number of subcarriers. Assume $|F|$ symbols to be transmitted $X_k$, with $k = 1, ..., |F|$. The $|F|$ symbols can be drawn from any of the constellation available, e.g., $X_k = \{-1,1\}$ for a BPSK modulation. The OFDM baseband modulated signal is given by

$$x(t) = \sum_{k=0}^{|\mathcal{F}|-1} X_k e^{2\pi k f_s t}, \; t \in [0, T_B] \tag{2}$$

where $f_s$ is the frequency spacing between subcarriers. The expression above represents an OFDM block of duration TB, where each symbol $X_k$ is transmitted on the $k^{th}$ subcarrier. If F is selected equal to 1, than the U-OFDM node will transmit in a single-carrier fashion, e.g., traditional narrowband modulations. An OFDM frame is defined as a set of consecutive OFDM blocks. To reduce the effect of multipath delay spread, a guard time TG is introduced between each OFDM block, such that multipath components from one block cannot interfere with the next block. The guard time could contain just silence, i.e., zero-padding (ZP), or a cyclic repetition of the block, i.e., cyclic prefixing (CP). ZP determines lower transmission power and simpler transmitter structure when compared to CP, but potentially affects the orthogonality of the subcarriers creating inter-carrier-interference (ICI). In the following, ZP is assumed unless otherwise specified. The expression in (2) becomes:

$$x(t) = \sum_{k=0}^{|\mathcal{F}|-1} X_k e^{2\pi k f_s t} g(t), \ t \in [0, T_c] \qquad (3)$$

where $T_c = T_B + T_G$ is the chip time, and $g(t)$ represents the ZP operation

$$g(t) = \begin{cases} 1 & t \in [0, T_B] \\ 0 & otherwise \end{cases} \qquad (4)$$

The resulting data rate [*bit/s*] is expressed as:

$$R = \frac{|\mathcal{F}| \log_2(M)}{T_c}, \qquad (5)$$

where M is the modulation rate, e.g., 1 for binary phase shift keying (BPSK), that is obtained as $2^N$, N being the number of bits conveyed per symbol.

The passband transmitted signal is obtained up-converting the baseband signal to a carrier frequency $f_c$.

$$s(t) = \text{Re} \left\{ \left[ \sum_{k=0}^{|\mathcal{F}|-1} X_k e^{2\pi k f_s t} g(t) \right] e^{-2\pi f_c t} \right\}, t \in [0, T_c] \quad (6)$$

Note that by assuming a sampling interval $T_s$, i.e., $t = nT_s$, and selecting the minimum frequency spacing between subcarriers that keeps orthogonality, i.e., $f_s = 1/nT_s$, the expression in (6), except for a constant, represents an N-point inverse discrete Fourier transform (IDFT) of the $X_k$ sequence. When $N$ is a power of two, the IDFT operation can be efficiently implemented using inverse fast-Fourier-transform (IFFT) algorithms.

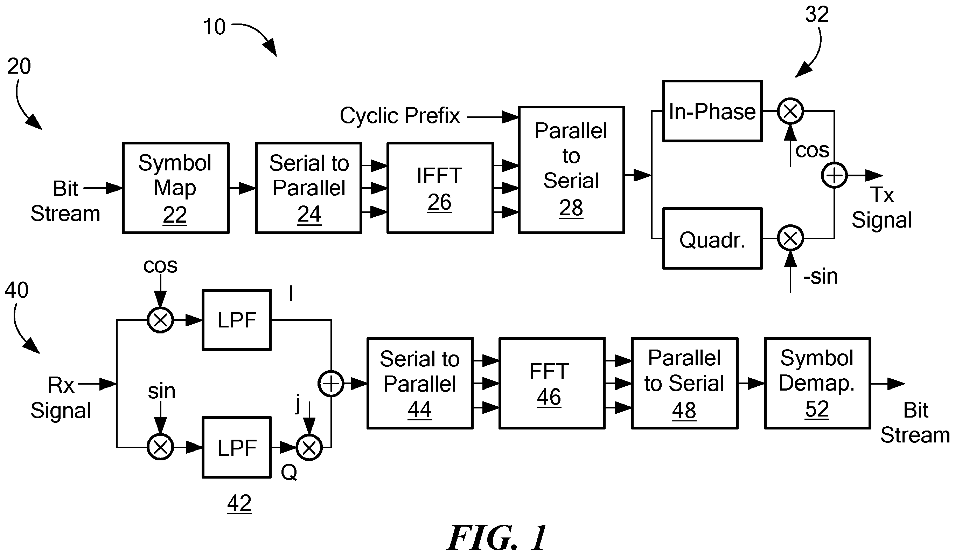

Fig. 1 shows a block diagram of an OFDM signal generator 10 with an encoder 20 and decoder 40. In the encoder 20, the bit stream is mapped into a conventional modulation constellation, e.g., M-PSK and M-QAM, at symbol mapper module 22. The serial symbol stream is converted into a parallel stream at serial-to-parallel converter 24 and fed into an TFFT module 26 that outputs the symbol representation in the frequency domain. The frequency domain samples are then converted into a serial stream, and a cyclic prefix (or the zero padding) is interleaved at the beginning of each IFFT block at parallel-to-serial converter 28. The resulting signal is up-converted to the carrier frequency $f_c$ at up-converter module 32 and transmitted. At the receiver side, the decoder 40 down-converts the received signal to baseband at down-converter module 42, and low-pass-filters it. The baseband filtered signal is transformed into a parallel stream at serial-to-parallel converter 44, fed into a fast-Fourier-transform (FFT) module 46, and re-serialized at parallel-to-serial converter 48 after discarding the cyclic prefix. The resulting symbol stream is then demapped at symbol demapper module 52 to obtain the received bit stream.

**Adaptive Subcarrier Frequency-Hopping.** In some embodiments, U-OFDM can use an adaptive subcarrier frequency-hopping that allows each transmitter to send symbols only in a subset of the available subcarriers, i.e., set of occupied subcarriers $F_O$, leaving the rest empty, i.e., set of null subcarriers $F_N$. The set of occupied subcarriers is selected randomly, and it changes in each consecutive block, according to a pseudo-random frequency-hopping sequence (FHS), i.e., a sequence generated by seeding a random number generator with the transmitter unique ID. The number of occupied subcarriers for the $i^{th}$ transmitter $N_{f,i} = |F_{o,i}|$ can be adaptively regulated between 1 and $N_{f,max} = |F|$, and is constant within an OFDM frame. Moreover, the sets of occupied and null subcarriers satisfy $= F_N \cup F_o$. The baseband OFDM block for the $i^{th}$ transmitter can be rewritten as

$$x_i(t) = \sum_{k \in \mathcal{F}_{o,i}} X_k e^{2\pi k f_s t} g(t), \ t \in [0, T_c] \qquad (7)$$

Since each subcarrier carries one symbol per block the resulting data rate becomes:

$$R(|\mathcal{F}_o|) = \frac{N_f \log_2(M)}{T_c}. \qquad (8)$$

By regulating the number of occupied subcarriers, $N_f$, a transmitter can adapt its data rate based on the level of occupancy of the frequency spectrum. The transmitter can also adapt $N_f$ based on the estimated coherence bandwidth of the channel. By pseudo-randomly selecting the set of subcarriers in each OFDM block, the probability that communications from different transmitters completely overlap can be lowered. Overlapping subcarriers produce subcarrier collisions, thus potential symbol detection errors. Ideally, increasing the number of available subcarriers $N_{f,max}$ may allow more transmitters to communicate in the same channel with lower probability of subcarrier collisions. However, $N_{f,max}$ is limited by the total available bandwidth B, which is limited because of the ultrasonic transducer characteristics, and by the computational power of the transmitters, i.e., the larger is $N_{f,max}$ the higher is the computational complexity to process the digital OFDM signals. Finally, subcarrier frequency-hopping also mitigates the effect of frequency-selective and fast fading in the intra-body channel caused by the presence of scatters and reflectors, as discussed above. Since the channel attenuates individually each subcarrier, the transmission performance highly fluctuates across different subcarriers and consequent OFDM blocks. By pseudo-randomly selecting the set of occupied subcarrier in each OFDM block a transmitter can average the fading effect.

It will be appreciated that in some embodiments, the U-OFDM signal generator can use subcarriers at fixed frequencies rather than at randomly selected subcarriers.

**Adaptive Time-Hopping.** Since subcarriers $N_f$ cannot be increased indefinitely, i.e., the probability of subcarrier collision cannot be lowered indefinitely, the adaptive subcarrier frequency-hopping may not be sufficient in heavy-load scenarios. For this reason, in some embodiments, U-OFDM can also leverage an adaptive time-hopping scheme that spreads in time OFDM blocks to further lower the probability of subcarrier collisions. We consider a slotted time divided in chips of duration $T_c$, with chips organized in frames of duration $T_f = N_h \cdot T_c$, where $N_h$ is the number of chips per frame. Each transmitter can send one OFDM block in one chip per frame, and determines in which chip to transmit based on a pseudo-random time hopping sequence (THS), i.e., a sequence generated by seeding a random number generator with the transmitter's unique ID. The baseband OFDM block for the $i^{th}$ transmitter and the $j^{th}$ OFDM block can be rewritten as

$$x_i(t, j) = \sum_{k \in \mathcal{F}_o^{(i)}} X_k e^{2\pi k f_s t} g(t) h(t), \ t \in [0, T_f] \qquad (9)$$

The function $h(t)$ represents the time-hopping spreading operation:

$$h(t) = \begin{cases} 1 & if \ c_{j,i} \\ 0 & otherwise \end{cases} \qquad (10)$$

where $\{c_{j,i}\}$ is the time hopping sequence of the $i^{th}$ source, with $0 \le c_{j,i} \le N_h - 1$. The resulting data rate becomes:

$$R(N_f, N_h) = \frac{N_f \log_2(M)}{T_f} = \frac{N_f \log_2(M)}{T_c N_h}. \qquad (11)$$

By regulating the time-hopping frame length $N_h$, i.e., the average inter-block time, a transmitter can adapt its data rate, and as a consequence modify the average radiated power and therefore the level of interference generated to other ongoing communications. It can be observed that an individual user (transmitter) has little incentive to increase its frame size, since that results in a lower achievable data rate, without any major benefit for the user itself (since the level of interference perceived depends primarily on the frame length of the other users, and not on its own). However, a longer time frame reduces the interference generated to the other users. Therefore, selfish/greedy frame adaptation strategies do not work well in this context and cooperative strategies are preferred.

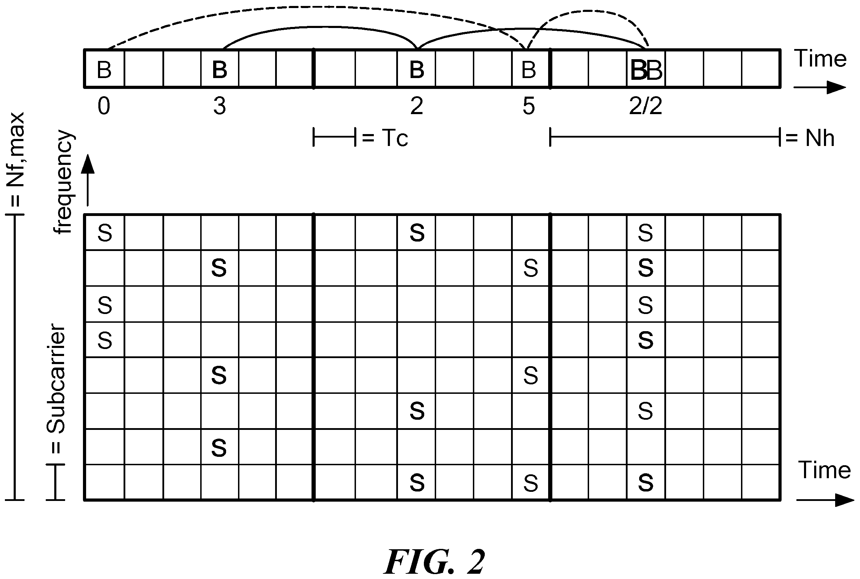

Fig. 2 shows an example of time-hopping strategy (top), and a combined frequency- and time-hopping strategy (bottom). In the pure time-hopping strategy, consider two users with $N_{h,1} = N_{h,2} = 5$, transmitting one block B per time-hopping frame, and using time hopping sequences $TH_1 = \{3, 2, 2\}$ and $TH_2 = \{0, 5, 2\}$. Since the two users select the same time chip in the third time-hopping frame, a collision between the two blocks occurs. This example can be extended by considering a combined frequency- and time-hopping strategy. Assume $N_{f,1} = N_{f2} = 3$ and $N_{f,max} = 8$. The two users transmit one symbol S per subcarrier per time-hopping frame. It can be observed that, although both users select the same subcarrier in their set of occupied subcarriers in the second time-hopping frame, the time-diversity introduced by the time-hopping avoids the collision between the two symbols. Similarly, in the third time-hopping frame the collision in time is avoided by leveraging of the frequency-diversity introduced by the frequency-hopping strategy.

It will be appreciated that is some embodiments, the U-OFDM signal generator can use fixed time chips with a time frame. In some embodiments, the time frame can comprise a variable number of time chips, with a minimum of one time chip (i.e., no time spreading). In some embodiments, an adaptive time-hopping scheme can be used alone, i.e., without an adaptive subcarrier frequency hopping scheme, or with a fixed frequency subcarrier scheme.

**Adaptive Channel Coding.** Since time and frequency hopping sequences are pseudo-randomly generated, collisions can still occur. In some embodiments, to mitigate the effect of mutual interference from co-located devices, U-OFDM can implement an adaptive channel coding that dynamically regulates the coding rate to adapt to channel conditions and interference level. In U-OFDM, coding adaptation is performed at the subcarrier level, i.e., in each sub-carrier the coding rate is individually and independently adapted to combat the effect of the channel distortions that occurs in that specific subcarrier. One embodiment uses forward error correction (FEC) functionality based on Reed-Solomon (RS) codes. RS codes are non-binary cyclic linear block error-correcting codes used in data storage and data transmission systems that have strong capability to correct both random and burst errors. An RS code can be denoted as $RS(s,n,k)$, where s is the symbol size in bits, $n$ is the block length and $k$ is the message length, with $k < n$. An RS encoder takes $k$ information symbols and adds $t$ parity symbols to make an $n$ symbol block. Therefore, there are $t = n - k$ overhead symbols. On the other hand, an RS decoder is able to decode the received n-symbol block, and can correct up to $t/2$ data symbols that may contain potential errors due to the channel fluctuation or collisions with interfering packets. The RS coding rate $r_c$ can be defined as the ratio between the message length and the block length, i.e., $r_c = k/n$. Since the coding operation introduces overhead symbols, the information rate is further reduced by a factor $1/r_c$, i.e.,

$$R(N_f, N_h, r_c) = \frac{r_c N_f \log_2(M)}{T_c N_h}. \qquad (12)$$

while the energy required for transmitting one bit is increased by a factor $1/r_c$. Note that there is a tradeoff between robustness to multi-user interference (which increases with lower coding rate), and energy consumption and information rate.

Alternative FEC technologies can also be used, for example convolutional codes that work on bit or symbol streams of arbitrary length and can be efficiently decoded with the Viterbi algorithm.

**Adaptive Modulation.** In some embodiments, adaptive modulation techniques can be used, which comprise adapting the modulation scheme in use to the channel condition to mitigate the effect of frequency-selective and fast fading and regulate the transmission rate. In U-OFDMA, adaptive modulation techniques can operate at the subcarrier level, at the block level or at the frame level. At the subcarrier level, different modulation schemes are selected for individual OFDM subcarriers in each OFDM block, to employ higher order modulations on subcarriers with high signal-to-noise ratio (SNR), lower order modulations on subcarriers with lower SNR, and no transmission on subcarriers with very low SNR. At the block and frame level, a single modulation scheme is used in all the subcarriers, and it can be changed every OFDM block or frame, respectively, based on the average SNR in all the subcarriers.

Frame and block level adaptation offer lower complexity when compared to subcarrier level adaptive modulation techniques, and in some embodiments, they can be a preferred choice when hardware resources are a constraint in miniaturized implantable devices. However, subcarrier level adaptive modulation techniques offer higher granularity that can be needed to achieve a desired communication quality of service in highly frequency selective channels. Section 3 below discusses joint dynamic adaptation of instantaneous power, number of occupied subcarriers, time-hopping frame length, FEC coding rate and modulation. Finally, since the modulation can vary between OFDM frames, the information rate is now also a function of the modulation rate $M$, i.e., $R(N_f, N_h, r_c, M)$.

**Frame Synchronization.** At the receiver, OFDM frame synchronization and "time hopping" synchronization must be performed to properly decode the received signal. Frame synchronization comprises finding the correct time instant corresponding to the start of an incoming packet at the receiver, and is achieved in two steps. First, an energy collection approach identifies any incoming frame, i.e., coarse synchronization. Once a frame is detected, the receiver performs a fine synchronization operation that identifies the exact starting point of the packet. Fine synchronization is achieved by correlating the received signal with a local copy of the preamble, i.e., an *a priori* known sequence that precedes each OFDM frame. After correlating the received signal and the expected signal, the receiver identifies the starting point of the packet as the time instant where the correlation is maximized. The second step comprises finding the time-hopping sequence to hop chip-by-chip to find the transmitted OFDM blocks. This can be achieved by seeding the random generator with the same seed used by the transmitter, and therefore generating the same pseudo-random time-hopping sequence.

U-OFDM can use as a preamble two different sequences, i.e., a pseudo noise (PN)-sequence and a chirp sequence. The former is a binary sequence with sharp autocorrelation peak and low cross-correlation peaks, that can be deterministically generated. Because of their desirable correlation characteristics, PN-sequences have strong resilience to multipath, and are well suited for ultrasonic intra-body channel, where reflections and scattering strongly affect the signal propagation, as discussed above in Section 1. The chirp sequence is a sinusoidal waveform whose frequency varies from an initial frequency $f_0$ to a final frequency $f_1$ within a certain time T. Chirps have been used in radars due to their good autocorrelation and robustness against Doppler effect. In fact, a frequency-shifted chirp still correlates well with the original chirp, although with lower amplitude and time-shifted peak. This characteristic makes chirp synchronization desirable in ultrasonic intra-body channels under severe Doppler effect conditions as experienced, for example, by an ingestible pill-sized camera moving in the digestive tract of the patient. The price paid for the Doppler robustness is higher cross-correlation peaks compared to PN-sequences that result in lower resilience to multipath.

**Channel Estimation and Equalization.** As discussed in Section 1, ultrasonic intra-body communications are affected by multipath and Doppler spread, leading to frequency selectivity. Since by using OFDM the symbol duration in each subcarrier is long compared to channel spread, inter-symbol interference (ISI) can be neglected in each subcarrier. However, the OFDM receiver is strongly limited by the inter-channel interference (ICI) due to fast channel variations within each OFDM symbol, especially if a ZP scheme is used. In some embodiments, channel estimation and equalization functionalities can be used to allow estimating the channel impulse response (CIR) and mitigating the distortion produced by the channel.

U-OFDM can implement both training-based and pilot-tone-based channel estimation. The training-based approach requires the presence of a training sequence known *a priori* in the transmitted packet, e.g., the synchronization preamble sequence, discussed in Section 2, to estimate the CIR. By correlating the output of the channel, i.e., the received signal, with the input, i.e., the known preamble sequence, an estimate of the time-domain CIR can be obtained. The pilot-tone-based approach estimates the channel for each OFDM block by leveraging a sequence of pilot symbols known *a priori* carried by a predefined group of subcarriers, i.e., pilot-subcarriers. This approach is suited for transmissions in channels that exhibit high time-variation, and therefore require estimating the CIR in each OFDM block. U-OFDM uses the CIR estimate for equalization, e.g., zero-forcing (ZF) or maximum-ratio combining (MRC), that aims to minimize the ICI signal distortion produced by the channel. Finally, to further reduce the ICI distortion, frequency-offset estimation due to Doppler effect is performed by leveraging the null subcarriers.

**Signal to Interference-plus-Noise Ratio.** The signal to interference-plus-noise ratio (SINR) at the receiver of link i averaged on all the occupied subcarriers and on a time-hopping frame length is defined as

$$\mathrm{SINR}_i(\mathbf{P}, \mathbf{N_f}, \mathbf{N_h}) = \sum_j^{N_{f,i}} \frac{P_i^{(j)} g_{i,i}^{(j)} N_{h,i} T_c}{\eta^{(j)} + T_c \sum_{k \in \mathcal{I}_i} \frac{N_{f,k}}{N_{f,i}} P_k^{(j)} g_{k,i}^{(j)}}, \qquad (13)$$

where $P_i^{(j)}$ is the instantaneous power emitted by the $i^{th}$ transmitter on the $j^{th}$ subcarrier, $g_{i,k}^{(j)}$ is the path gain between the $j^{th}$ transmitter and the $k^{th}$ receiver on the $j^{th}$ subcarrier, and $\eta^{(j)}$ represents background noise energy on the $j^{th}$ subcarrier. The set $I_i$ represents the set of links whose transmitter interferes with the receiver of link i. Note that, the expression in (13) depends on the array of instantaneous power, time-hopping frame length and number of occupied subcarrier of all the ongoing communications in the network, i.e., $P, N_h, N_f$, whose $i^{th}$ elements are $P_i, N_{h,i}$ and $N_{f,i}$, respectively. The term $N_{f,k}/N_{f,i}$ is the relative number of occupied carriers between the $k^{th}$ interferer and the $i^{th}$ user. This ratio scales/weights the interference effect of the $k^{th}$ transmitter over the communication of the $i^{th}$ user. When different links use different frame lengths, (13) becomes

$$\mathrm{SINR}_i(\mathbf{P}, \mathbf{N_f}, \mathbf{N_h}) = \sum_{j}^{N_{f,i}} \frac{P_i^{(j)} g_{i,i}^{(j)} N_{h,i} T_c}{\eta^{(j)} + T_c \sum_{k \in \mathcal{I}_i} \frac{N_{f,k}}{N_{f,i}} \frac{N_{h,i}}{N_{h,k}} P_k^{(j)} g_{k,i}^{(j)}},$$

$$(14)$$

The term $N_{h,i}/N_{h,k}$ accounts for the level of interference generated by each interferer $k$ to the receiver of link $i$, i.e., the number of pulses transmitted by the $k^{th}$ transmitter during the time frame of the $i^{th}$ user.

Note that when the node of interest increases (decreases) its frame length, $N_{h,i}$ while the other nodes do not, no variation is expected in the SINR (there is in fact a slight increase (decrease) in the SINR, which can be neglected under high SNR conditions, $\eta \ll \Sigma_{k \in I_i} P_k g_{ki}$). Finally, when the frame length of the interfering nodes is increased (decreased), the SINR increases (decreases). On the other hand, when the interfering nodes increase (decrease) their number of occupied subcarriers, the SINR decreases (increases), since the probability of subcarrier collision increases (decreases).

As can be observed in (14), modulation and FEC coding rate do not affect the average SNR measured at the receiver. Instead, these two parameters affect the relation between bit-error-rate (BER) experienced at the receiver and SINR. Lower FEC coding rate means more overhead symbols, which potentially lower the BER after decoding. Thus, for a given minimum BER threshold, lower FEC coding rates require lower minimum SINR. On the other end, for a given BER requirement, increasing the modulation order requires a higher minimum SINR level to achieve that BER. For these reasons, the minimum SINR requirement, i.e., $\mathrm{SINR}_{min}$, is expressed as a function of the FEC coding rate and the modulation order, i.e., $\mathrm{SINR}_{min}(r_c, M)$ .

### 3. MAC and Rate Adaptation

In some embodiments, U-OFDM medium access control protocol and rate adaptation schemes can be provided. Based on the discussion so far, there is a tradeoff between (i) resilience to interference and channel errors, (ii) achievable information rate, and (iii) energy efficiency. Thus, medium access control and rate adaptation strategies can be provided that find optimal operating points along efficiency-reliability tradeoffs. Rate-maximizing adaptation strategies are discussed in Section 3.1. Energy-minimizing strategies are discussed in Section 3.2.

### 3.1 Distributed Rate-Maximizing Adaptation

The objective of the rate-adaptation scheme under consideration is to let each active communication maximize its transmission rate by optimally selecting the instantaneous power, the number of occupied subcarrier, the time-hopping frame length, the FEC coding rate and modulation, based on the current level of interference and channel quality measured at the receiver and on the level of interference generated by the transmitter to the other ongoing communications. A decentralized ultrasonic intra-body area network is considered, with N being the set of |N| existing connections. Note that there are no predefined constraints on the number of simultaneous connections |N|. Denote by $N_{h,max}$, $r_{c,max}$, $Mmax$ and $P_{max}$, the maximum time-hopping frame length, the maximum coding rate, the maximum modulation rate and the maximum instantaneous power supported, respectively. Thus

$$0 < N_{f,i} \leq N_{f,max}, \quad \forall i \in \mathcal{N}, \mathrm{N}_f \in \mathbb{N}, \qquad (15)$$

$$0 < N_{h,i} \leq N_{h,max}, \quad \forall i \in \mathcal{N}, \mathrm{N}_h \in \mathbb{N}, \qquad (16)$$

$$r_{c,i} \in \{r_{c,i}, r_{c,2}, \dots, r_{c,max}\} \ \forall i \in \mathcal{N}, \qquad (17)$$

$$M_i \in \{M1, M2, \dots, Mmax\} \ \forall i \in \mathcal{N}, \qquad (18)$$

$$0 \geq P_i \leq P_{max} \ \forall i \in \mathcal{N}, \qquad (19)$$

where N is the set of natural numbers. According to the transmission scheme discussed in Section 2, each node i transmits at a rate $R^{(i)}$ expressed as in (12) and each receiver experiences an SINR expressed as in (14). Each node has a minimum data rate requirement,

$$R_i\,(N_{f,i},\ N_{h,i},\ r_{c,i},\ M_i)\ \geq R_{min}, \tag{20}$$

and a minimum SINR requirement,

$$\mathrm{SINR}_i(P_i,\ N_{f,i},\ N_{h,i})\geq \mathrm{SINR}_{min}(r_{c,i},\ M_i) \tag{21}$$

The receiver is in charge of estimating interference and finding the instantaneous power, number of occupied subcarriers, time-hopping frame length, FEC coding rate and modulation that maximize the system performance. Accordingly, denote instantaneous power, the number of occupied subcarrier, the time-hopping frame length, the FEC coding rate and modulation selected by the receiver of the connection $r$, as $P_r, N_{f,r}, N_{h,r}, r_c, M_r$. The objective of each user is to locally optimize the information rate of the connection by solving the following problem:
find

$$P_r,\ N_{f,r},\ N_{h,r},\ r_{c,r},\ M_r \tag{22}$$

that maximize

$$R_r,(N_{f,r},\ N_{h,r},\ r_{c,r},\ M_r) \tag{23}$$

subject to

$$R_r,(N_{f,r},\ N_{h,r},\ r_{c,r},\ M_r)\ \geq R_{min} \tag{24}$$

$$\mathrm{SINR}_r(P_r,\ N_{f,r},\ N_{h,r})\geq \mathrm{SINR}_{r,min}(r_{c,r},\ M_r) \tag{25}$$

$$\mathrm{SINR}_i(P_i,\ N_{f,i},\ N_{h,i})\geq \mathrm{SINR}_{i,min}(r_{c,i},\ M_i)\quad \forall\,i\in \mathcal{I}_r, \tag{26}$$

where $I_r$ is the set of the connections interfering with the $r^{th}$ connection. The constraints on the maximum frame and code length in (15), (16), (17), (18) and (19) are also implicitly considered.

### 3.2 Distributed Energy-Minimizing Rate Adaptation

Rate adaptation has the objective of reducing the energy consumption of U-OFDM. Section 2 mentioned that adaptive frequency and time hopping techniques, adaptive FEC coding and adaptive modulation affect the energy consumption of the transmitting device. For this reason, energy-related metrics are introduced that make the dependence of the energy consumption on number of occupied subcarriers, time-hopping frame length, FEC coding and modulation explicit.
$E_b$, the energy per bit is defined as:

$$E_b = P{\cdot}T_c/(r_c{\cdot}N_f{\cdot}log_2(M)) \tag{27}$$

$E_s$, the average power radiated per second is defined as:

$$E_s = P/(N_h). \tag{28}$$

The energy per bit is a function of the inverse of the FEC coding rate, number of occupied subcarriers and number of bits transmitted per symbol. Higher coding rate, number of occupied subcarrier and modulation order decrease the energy consumption. The average power emitted per second is a function of the inverse of the time-hopping frame length and hence of the number of OFDM blocks transmitted per second.

**Energy-minimizing Rate Adaptation.** Based on this model, a rate adaptation strategy is provided where the objective is to minimize (i) the energy per bit, $E_b$, or (ii) the average energy emitted per second $E_s$. The problem can be cast as finding the optimal frame length and the optimal spreading code length that minimize $E_b$ (and/or $E_s$) while meeting the minimum SINR constraints and keeping the data rate over a given threshold. The problem is formally expressed below.

Find $P_r, N_{f,r}, N_{h,r}, r_{c,r}, M_r$

that minimizes $E_b(P_r, r_{c,r}, N_{f,r}, M_r)$ (or $E_s(N_{h,r})$)

subject to Equations (24, (25) and (26).

## 3.3 Medium Access Control Protocol

In some embodiments of U-OFDM, distributed medium access control coordination can be achieved by exchanging information on logical control channels, while data packets are transmitted over logical data channels. Unicast transmissions between a transmitter *TX* and a receiver *RX* are considered, as follows.

When *TX* needs to transmit a packet, it first needs to reserve a dedicated channel to *RX.* The connection is opened through the common control channel using a two-way handshake procedure. In U-OFDM the control channel can be implemented using two different alternative approaches, fixed control channel and random control channel. In the fixed control channel approach, a fixed number of preassigned subcarriers are allocated to transmit and receive control information. In the control subcarriers the communication follows a unique time-hopping sequence known and shared by all network devices. All the nodes listen to the fixed control channel and wait for a request from a transmitting node. The control channel is accessed through a contention phase.

In the random control channel approach, control channel is implemented in a frequency-hopping fashion, i.e., the control channel subcarrier allocation changes pseudo-randomly in time. Synchronization between the transmitting and receiving nodes is possible by guaranteeing that the transmitter use all the channels in a fixed period of time, so that the receiver can then find the transmitter channel by picking a random channel and listening for valid data on that channel.

In the two-way handshake procedure, *TX* sends a Request-to-Transmit (R2T) packet to *RX,* which contains its own ID. If *RX* is idle, a Clear-to-Transmit (C2T) control packet is sent back to *TX.* In case of failure and consequent timer expiration, *TX* will attempt a new transmission after a random backoff time, for a maximum of $N_R$ times. After receiving the C2T packet, the transmitter switches to a dedicated channel by computing its own frequency- and time-hopping sequence by seeding a pseudo-random sequence generator with its own ID. As a consequence, both *TX* and *RX* leave the common channel and switch to a dedicated channel. The receiver *RX* computes the optimal transmission strategy, i.e., number of occupied subcarrier, time-hopping frame length, FEC coding rate and modulation, as discussed in Section 2. This information is piggybacked into ACK or NACK packets.

Once the communication has been established, *RX* does not leave the common control channel. Instead, it keeps "listening" to both the dedicated and common control channels at the same time. In the dedicated control channel, *RX* sends to *TX* the optimal strategy information to be used for the next transmission. In the common control channel, *RX* exchanges with other co-located receivers information on the level of tolerable interference.

## 3.4 **Network Configuration**

U-OFDM can internetwork implantable devices in master/slave (M/S) or peer-to-peer (P2P) configurations. Both configurations can coexist in the same intra-body network, referred to as hybrid configurations.

**Master-Slave Configuration.** In the M/S configuration, one node takes the role of master, i.e., network coordinator, while the remaining nodes operate as slaves. In this scenario, the network control is concentrated on a master node. Network access of the slave node is deterministically regulated through a polling mechanism, e.g., the master node has complete control over channel access, while each slave node is granted access to the medium in a round-robin fashion.

**Peer-to-Peer Configuration.** In the P2P configuration, all the network nodes are treated as peers. In this configuration, network access can be regulated as discussed in Section 3.3 through fixed or random control channel.

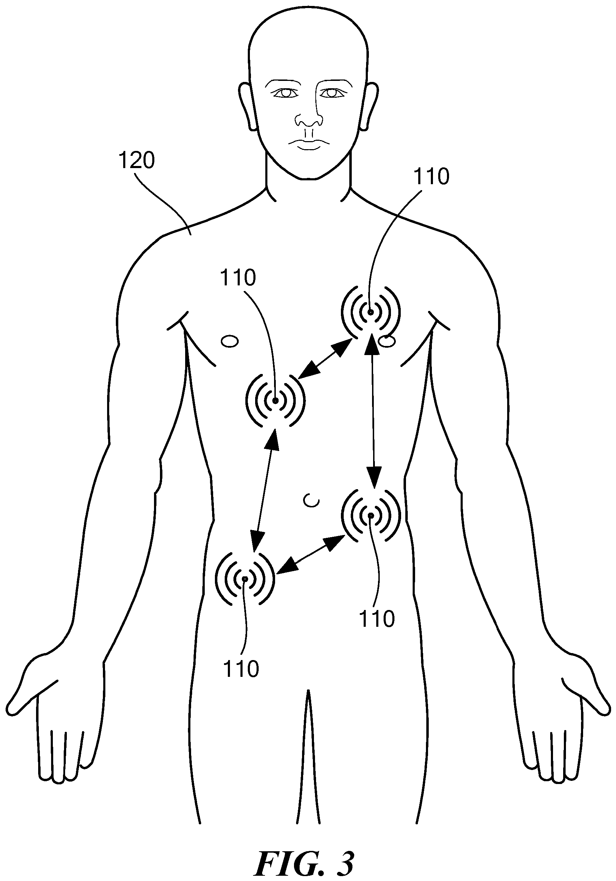

The communication system and method for transmitting data ultrasonically through biological tissue can be advantageously implemented among a network comprising a plurality of nodes 110 in which at least a portion of the nodes are implantable within a body 120. See Fig. 3. In some embodiments, at least one of the implantable nodes, a first node, comprises an ultrasonic transducer and a transmitter, and a second node, which can be implantable within the body or disposable outside the body, comprises an ultrasonic receiver. The transmitter at the first node includes an orthogonal frequency division multiplex (OFDM) signal generator operative to encode an input information bit stream on orthogonal subcarriers for transmission as an ultrasonic signal through the body to the ultrasonic receiver at the second node. The ultrasonic receiver at the second node is operative to decode the ultrasonic signal received from the first node to recover the information bit stream. In some embodiments, all of the nodes are implantable in a body.

Each node can include a combination of hardware, software, and/or firmware that allows the system to perform the various tasks as described herein. The nodes can be implemented as microprocessor-based computing devices, microcontroller-based computing devices, and the like. The computing devices can include one or more processors and memory that cooperate with an operating system to provide basic and support functionality for an applications layer and other processing tasks. Various types of processing technology can be used, including a single processor or multiple processors, a central processing unit (CPU), multicore processors, parallel processors, or distributed processors. Additional specialized processing resources, such as mathematical processing capabilities, can be provided to perform certain processing tasks. Other hardware components and devices can interface with the computing device. As used herein, the term "transceiver" can include one or more devices that both transmit and receive signals, whether sharing common circuitry, housing, or a circuit board, or whether distributed over separated circuitry, housings, or circuit boards, and can include a transmitter-receiver.

The computing device includes memory or storage, which can be accessed by a system bus or in any other manner. Memory can store control logic, instructions, and/or data. Memory can include transitory memory, such as cache memory, random access memory (RAM), static random access memory (SRAM), main memory, dynamic random access memory (DRAM), and memristor memory cells. Memory can include storage for firmware or microcode, such as programmable read only memory (PROM) and erasable programmable read only memory (EPROM). Memory can include non-transitory or nonvolatile or persistent memory such as memory chips and memristor memory cells. Any other type of tangible, non-transitory storage that can provide instructions and/or data to a processor can be used in these embodiments.

The communication system and method can be used in a variety of applications that require transmission of data through biological tissue. For example, the system and method can be used with implantable sensors, such as cardiac rhythm monitors, pulse monitors, blood pressure sensors, glucose sensors, drug pump monitors, motion sensors, gyroscopes, accelerometers, sleep sensors, REM sleep duration sensors, still cameras, or video cameras, to transmit physiological data that is captured by the implantable sensors to a gateway outside of the body or to other implanted devices within the body. The system can be used to communicate actuation commands to and obtain data from implantable devices such as drug delivery systems or drug pumps, heart stimulators, pacemakers, neuromuscular electrical stimulators, and bone growth stimulators. For example, the system and method can be used to obtain data from a glucose monitor in a diabetic patient and to communicate instructions to and obtain data from a miniaturized, under-the-skin insulin pump. As another example, the system and method can be used with pill-sized ingestible cameras that are used to monitor the digestive tract of a patient. The system can be used with a human body or with a non-human animal body.

In certain embodiments, a node can include a sensor for one or more biomolecules. Examples of such biomolecules include peptides, oligopeptides, polypeptides, proteins, glycoproteins, antibodies, antigens, nucleic acids, nucleotides, oligonucleotides, polynucleotides, sugars, disaccharides, trisaccharides, oligosaccharides, polysaccharides, lipids, glycolipids, proteolipids, cytokines, hormones, neurotransmitters, metabolites, glycosaminoglycans, and proteoglycans. In certain embodiments, a node can include a sensor for one or more pharmaceutical agents or pharmaceutical formulation ingredients. In certain embodiments a node can include a sensor for a dissolved gas or ion, or for pH, ionic strength, or osmolarity.

As used herein, "consisting essentially of" allows the inclusion of materials or steps that do not materially affect the basic and novel characteristics of the claim. Any recitation herein of the term "comprising," particularly in a description of components of a composition or in a description of elements of a device, can be exchanged with "consisting essentially of" or "consisting of."

**Claims**

1. A system for transmitting data ultrasonically through biological tissue comprising:

   a network comprising a plurality of nodes, at least a portion of the nodes implantable within a body; and
   a first node implantable in the body and comprising an ultrasonic transducer and a transmitter, and a second node comprising an ultrasonic receiver;
   the system **characterized in that** the transmitter at the first node includes an orthogonal frequency division multiplex (OFDM) signal generator operative to encode an input information bit stream on orthogonal subcarriers for transmission as an ultrasonic signal through the body to the ultrasonic receiver at the second node; and
   that the ultrasonic receiver at the second node is operative to decode the ultrasonic signal received from the first node to recover the information bit stream.

2. The system of claim 1, wherein the OFDM signal generator is operative to generate a baseband modulated signal as a sum over a number of subcarriers of the symbols to be transmitted as a function of a frequency spacing between the subcarriers for a time block of a given duration.

3. The system of claim 2, wherein the OFDM signal generator is operative to introduce a guard time between time blocks, and preferably wherein the guard time comprises silence or a repetition of the time block.

4. The system of claim 2, further comprising a symbol mapper to map the bit stream into a constellation of a modulation scheme, and preferably wherein the modulation scheme comprises M-phase-shift-keying or M-quadrature-amplitude-modulation.

5. The system of claim 1, wherein the OFDM signal generator comprises:

   a serial to parallel convertor to convert the input information bit stream into a plurality of parallel data strings,
   an inverse Fourier transformer to generate a frequency domain representation of the input information bit stream,
   a parallel to serial converter for converting the frequency domain representation into a serial stream, and
   an up-converter to convert the signal to a carrier frequency for transmission through the biological tissue.

6. The system of claim 1, wherein the receiver comprises:

   a down-converter to convert the received signal to a baseband signal,
   a serial to parallel converter to convert the baseband signal into a plurality of parallel data strings,
   a Fourier transformer; and
   a parallel to serial converter to convert the parallel data strings into a serial data string.

7. The system of claim 1, wherein the OFDM signal generator is operative to send symbols on a set of occupied subcarriers comprising a subset of available subcarriers, and preferably one or more of:

   wherein the occupied subcarriers are fixed or selected randomly and change in consecutive blocks within a frame; and
   wherein the occupied subcarriers are selected by a pseudo-random frequency-hopping sequence generated by seeding a random number generator with an identification unique to the transmitter.

8. The system of claim 1, wherein the OFDM signal generator is operative to send symbols in blocks at fixed or randomly selected time chips within a time frame; and preferably one or more of:

   wherein the OFDM signal generator is operative to send symbols according to a pseudo-random time hopping sequence generated by seeding a random number generator with an identification unique to the transmitter; and
   wherein the receiver is operative to decode the ultrasonic signal at the receiver by seeding a random generator with the identification of the transmitter to generate the same pseudo-random time hopping sequence.

9. The system of claim 1, wherein the OFDM signal generator is operative to provide forward error correction, and preferably one or more of:

   wherein the forward error correction comprises adding $t$ parity symbols to $k$ information symbols to make an $n$ symbol block; and
   wherein the forward error correction comprises the addition of parity symbols using a block code or a convolutional code.

10. The system of claim 1, wherein the OFDM signal generator is operative to provide one or more modulation techniques at a subcarrier level, a block level, or a frame level, and preferably wherein the modulation technique is selected to optimize a data rate as a function of one or more of a number of occupied subcarriers, a number of time chips per time frame, an error correction coding rate, and a modulation rate.

11. The system of claim 1, wherein the receiver is operative to detect an incoming frame from the transmitter and to identify a starting point of a packet; and preferably one or more of:

wherein identifying the starting point comprises correlating the received ultrasonic signal with a local copy of a preamble preceding each OFDM frame; and

wherein the preamble comprises a pseudo noise sequence or a chirp sequence.

**12.** The system of claim 1, further comprising one or more of:

wherein the receiver is operative to determine a signal to interference-plus-noise ratio as a function of instantaneous power, time-hopping frame length, and number of occupied subcarriers;

wherein the receiver is operative to maximize a transmission rate between the transmitter and the receiver by selecting an instantaneous power, a number of occupied subcarriers, a time-hopping frame length, a forward error correction coding rate and a modulation rate based on a level of interference and channel quality measured at the receiver and on a level of interference generated by the receiver in communications to other nodes;

wherein the receiver is operative to determine an instantaneous power value, a number of occupied subcarriers, a time-hopping frame length, a forward error correction coding rate and a modulation rate that maximizes a data rate;

wherein the receiver is further operative to maximize the data rate subject to a signal to interference-plus-noise ratio per node being above a minimum value and a data rate per node being above a minimum value;

wherein the receiver is further operative to determine an energy rate, the energy rate comprising an energy per bit or an average power radiated per second; and

wherein the receiver is further operative to minimize the energy rate subject to a signal to interference-plus-noise ratio per node being above a minimum value and a data rate per node being above a minimum value.

**13.** The system of claim 1, wherein the transmitter is operative to open communication to a receiver on a common control channel using a two-way hand-shake procedure, and the transmitter is operative to transmit on a dedicated channel to the receiver a frequency-hopping sequence and a time-hopping sequence after receiving a clear-to-transmit signal from the receiver, and the receiver is operative to transmit to the transmitter an optimal transmission strategy; and preferably one or more of:

wherein the transmitter is operative to determine the frequency-hopping sequence and the time-hopping sequence by seeding a random number generator with an identification unique to the transmitter;

wherein the optimal transmission strategy comprises a number of occupied subcarriers, a time-hopping frame length, a forward error correction coding rate, and a modulation rate; and

wherein the receiver is operative to exchange information regarding a level of tolerable interference over the common control channel with other receiving nodes.

**14.** The system of claim 1, wherein the first node further comprises an ultrasonic receiver to decode an ultrasonic signal received from another node of the plurality of nodes; and preferably one or more of:

wherein the second node further comprises an ultrasonic transducer and a transmitter, the transmitter at the second node including an orthogonal frequency division multiplex (OFDM) signal generator operative to encode an input information bit stream on orthogonal subcarriers for transmission as an ultrasonic signal through the body to the first node or to another node of the plurality of nodes or to all of the nodes of the plurality of nodes; and

wherein the first implantable node further comprises one or both of a sensor operative to sense one or more biological parameters or an actuator.

**15.** A method for transmitting data ultrasonically through biological tissue comprising:

at a first node implanted in a body, providing a signal;

transmitting the signal through biological tissue; and

at a second node, receiving the signal;

**characterized in that** the signal is an information bit stream encoded on orthogonal subcarriers using an orthogonal frequency division multiplexing modulation scheme; and that the signal is decoded upon reception to recover the information bit stream;

and preferably further comprising one or more of:

encoding the information bit stream comprises generating a baseband modulated signal as a sum over a number of subcarriers of the symbols to be transmitted as a function of a frequency spacing between the subcarriers for a time block of a given duration;

sending symbols on a set of occupied subcarriers comprising a subset of available subcarriers, wherein the occupied subcarriers are fixed or selected randomly and change in consecutive blocks within a frame; sending symbols in blocks at fixed or randomly selected time chips within a time frame; providing forward error correction, wherein the forward error correction comprises the addition of parity symbols using a block code or a convolutional code; at the second node, detecting an incoming frame from the first node and identifying a starting point of a packet, comprising correlating the received ultrasonic signal with a local copy of a preamble preceding an OFDM frame, wherein the preamble comprises a pseudo noise sequence or a chirp sequence; and at the second node, maximizing a transmission rate between the transmitter and the receiver by selecting an instantaneous power, a number of occupied subcarriers, a time-hopping frame length, a forward error correction coding rate and a modulation rate based on a level of interference and channel quality measured at the receiver and on a level of interference generated by the second node in communications to other nodes.

**Patentansprüche**

1. System zur Ultraschallübertragung von Daten durch biologisches Gewebe, Folgendes umfassend:

   ein Netzwerk, das eine Vielzahl von Knoten umfasst, wobei mindestens ein Teil der Knoten in einem Körper implantierbar ist; und
   einen ersten Knoten, der im Körper implantierbar ist und einen Ultraschallwandler und einen Sender umfasst, und einen zweiten Knoten, der einen Ultraschallempfänger umfasst;
   wobei das System **dadurch gekennzeichnet ist, dass** der Sender am ersten Knoten einen orthogonalen Frequenzmultiplex(OFDM)-Signalgenerator beinhaltet, der dazu betrieben wird, einen Eingabeinformationsbitstrom auf orthogonalen Subträgern zur Übertragung als Ultraschallsignal durch den Körper an den Ultraschallempfänger am zweiten Knoten zu verschlüsseln; und
   dass der Ultraschallempfänger am zweiten Knoten dazu betrieben wird, das Ultraschallsignal, das vom ersten Knoten empfangen wird, zu entschlüsseln, um den Informationsbitstrom wiederzuerlangen.

2. System nach Anspruch 1, wobei der OFDM-Signalgenerator dazu betrieben wird, ein basisbandmoduliertes Signal als Summe über eine Anzahl von Unterträgern der zu übertragenden Symbole in Abhängigkeit einer Frequenzbeabstandung für einen Zeitblock einer gegebenen Dauer zwischen den Unterträgern zu generieren.

3. System nach Anspruch 2, wobei der OFDM-Signalgenerator dazu betrieben wird, eine Bewachungszeit zwischen Zeitblöcken einzuführen und wobei die Bewachungszeit vorzugsweise Stille oder eine Wiederholung des Zeitblocks umfasst.

4. System nach Anspruch 2, ferner umfassend einen Symbolabbilder zum Abbilden des Bitstroms in eine Konstellation eines Modulationsverfahrens, und wobei das Modulationsverfahren vorzugsweise eine M-Phasenumtastung- oder M-Quadraturampitudenmodulation umfasst.

5. System nach Anspruch 1, wobei der OFDM-Signalgenerator Folgendes umfasst:

   einen Parallel-Seriell-Umsetzer zum Konvertieren des Eingabeinformationsbitstroms in eine Vielzahl paralleler Datenketten,
   einen inversen Fourier-Transformator zum Generieren einer Frequenzdomänendarstellung des Eingabeinformationsbitstroms,
   einen Parallel-Seriell-Umsetzer zum Konvertieren der Frequenzdomänendarstellung in einen Serienstrom, und
   einen Aufwärtswandler zum Konvertieren des Signals in eine Trägerfrequenz zur Übertragung durch das biologische Gewebe.

6. System nach Anspruch 1, wobei der Empfänger Folgendes umfasst:

   einen Abwärtswandler zum Konvertieren des empfangenen Signals in ein Basisbandsignal,
   einen Seriell-Parallel-Umsetzer zum Konvertieren des Basisbandsignals in eine Vielzahl paralleler Datenketten,
   einen Fourier-Transformator; und
   einen Parallel-Seriell-Umsetzer zum Konvertieren der parallelen Datenketten in eine Seriendatenkette.

7.  System nach Anspruch 1, wobei der OFDM-Signalgenerator dazu betrieben wird, Symbole auf einem Satz besetzter Unterträger zu senden, die einen Teilsatz verfügbarer Unterträger umfassen, und vorzugsweise eines oder mehrere von Folgendem:

    wobei die besetzten Unterträger festgelegt oder zufällig ausgewählt sind und sich in nachfolgenden Blöcken innerhalb eines Frames ändern; und

    wobei die besetzten Unterträger durch eine pseudozufällige Frequenzsprungsequenz ausgewählt sind, die durch Seeden eines Zufallszahlgenerators mit einer für den Sender eindeutigen Kennung generiert wird.

8.  System nach Anspruch 1, wobei der OFDM-Signalgenerator dazu betrieben wird, Symbole in Blöcken zu festgelegten oder zufällig ausgewählten Zeitchips innerhalb eines Zeitrahmens zu senden; und vorzugsweise eines oder mehrere von Folgendem:

    wobei der OFDM-Signalgenerator dazu betrieben wird, Symbole gemäß einer pseudozufälligen Zeitsprungsequenz zu senden, die durch Seeden eines Zufallszahlgenerators mit einer für den Sender eindeutigen Kennung generiert wird; und

    wobei der Empfänger dazu betrieben wird, das Ultraschallsignal am Empfänger durch Seeden eines Zufallsgenerators mit der Kennung des Senders zu entschlüsseln, um dieselbe pseudozufällige Zeitsprungsequenz zu generieren.

9.  System nach Anspruch 1, wobei der OFDM-Signalgenerator dazu betrieben wird, eine Vorwärtsfehlerkorrektur bereitzustellen, und vorzugsweise eines oder mehrere von Folgendem:

    wobei die Vorwärtsfehlerkorrektur das Hinzufügen von $t$-Paritätssymbolen zu $k$-Informationssymbolen umfasst, um einen $n$-Symbolblock zu erstellen; und

    wobei die Vorwärtsfehlerkorrektur das Hinzufügen von Paritätssymbolen unter Verwendung eines Blockcodes oder eines Faltungscodes umfasst.

10. System nach Anspruch 1, wobei der OFDM-Signalgenerator dazu betrieben wird, eine oder mehrere Modulationstechniken auf einer Unterträgerebene, einer Blockebene oder einer Frameebene bereitzustellen, und wobei die Modulationstechnik vorzugsweise zum Optimieren einer Datenrate in Abhängigkeit einer oder mehrerer einer Anzahl besetzter Unterträger, einer Anzahl von Zeitchips pro Zeitrahmen, einer Fehlerkorrekturcodierungsrate und einer Modulationsrate ausgewählt ist.

11. System nach Anspruch 1, wobei der Empfänger dazu betrieben wird, einen eingehenden Frame vom Sender zu erfassen und als Ausgangspunkts eines Pakets zu identifizieren; und vorzugsweise eines oder mehrere von Folgendem:

    wobei das Identifizieren des Ausgangspunkts Korrelieren des empfangenen Ultraschallsignals mit einem lokalen Exemplar einer Präambel umfasst, die jedem OFDM-Frame vorausgeht; und

    wobei die Präambel eine Pseudogeräuschsequenz oder eine Chirp-Sequenz umfasst.

12. System nach Anspruch 1, ferner umfassend eines oder mehrere von Folgendem:

    wobei der Empfänger dazu betrieben wird, ein Verhältnis von Signal zu Störung-plus-Geräusch in Abhängigkeit von Augenblicksleistung, Zeitsprung-Framelänge und einer Anzahl belegter Unterträger zu bestimmen;

    wobei der Empfänger dazu betrieben wird, eine Übertragungsrate zwischen dem Sender und dem Empfänger durch Auswählen einer Augenblicksleistung, einer Anzahl belegter Unterträger, einer Zeitsprung-Framelänge, einer Vorwärtsfehlerkorrekturcodierungsrate und einer Modulationsrate auf Grundlage eines Störungsniveaus und einer Kanalqualität, die am Empfänger gemessen werden, und eines Störungsniveaus, das durch den Empfänger in Kommunikation mit anderen Knoten generiert wird, zu maximieren;

    wobei der Empfänger dazu betrieben wird, einen Augenblicksleistungswert, eine Anzahl belegter Unterträger, eine Zeitsprung-Framerate, eine Vorwärtsfehlerkorrekturcodierungsrate und eine Modulationsrate zu bestimmen, die eine Datenrate maximiert;

    wobei der Empfänger ferner dazu betrieben wird, die Datenrate vorbehaltlich dessen zu maximieren, dass ein Verhältnis von Signal zu Störung-plus-Geräusch pro Knoten über einem Höchstwert und eine Datenrate pro Knoten über einem Mindestwert liegt;

    wobei der Empfänger ferner dazu betrieben wird, eine Energierate zu bestimmen, wobei die Energierate eine Energie pro Bit oder eine pro Sekunde abgestrahlte Durchschnittsleistung umfasst; und wobei der Empfänger ferner dazu betrieben wird, die Energierate vorbehaltlich dessen zu minimieren, dass ein Verhältnis von Signal zu Störung-plus-Geräusch pro Knoten über einem Mindestwert und eine Datenrate pro Knoten über einem Mindestwert liegt.

13. System nach Anspruch 1, wobei der Sender dazu betrieben wird, die Kommunikation an einen Empfänger auf einem gemeinsamen Steuerkanal unter Verwendung eines Zweiwege-Handshake-Verfahrens zu eröffnen, und wobei der Sender dazu betrieben wird, auf einem dedizierten Kanal eine Frequenzsprungsequenz und eine Zeitsprungsequenz an den Empfänger zu übertragen, nachdem er vom Empfänger ein Sendebereit-Signal erhalten hat, und der Empfänger dazu betrieben wird, eine optimale Übertragungsstrategie an den Sender zu übertragen; und vorzugsweise eines oder mehrere von Folgendem:

wobei der Sender dazu betrieben wird, die Frequenzsprungsequenz und die Zeitsprungsequenz durch Seeden eines Zufallszahlgenerators mit einer für den Sender eindeutigen Kennung zu bestimmen;
wobei die optimale Übertragungsstrategie eine Anzahl belegter Unterträger, eine Zeitsprung-Framelänge, eine Vorwärtsfehlerkorrekturcodierungsrate und eine Modulationsrate umfasst; und
wobei der Empfänger dazu betrieben wird, Informationen in Bezug auf ein Niveau tolerierbarer Störung über den gemeinsamen Steuerkanal mit anderen empfangenden Knoten auszutauschen.

14. System nach Anspruch 1, wobei der erste Knoten ferner einen Ultraschallempfänger zum Entschlüsseln eines Ultraschallsignals umfasst, das von einem anderen Knoten in der Vielzahl von Knoten empfangen wird; und vorzugsweise eines oder mehrere von Folgendem:

wobei der zweite Knoten ferner einen Ultraschallwandler und einen Sender umfasst, wobei der Sender am zweiten Knoten einen orthogonalen Frequenzmultiplex(OFDM)-Signalgenerator beinhaltet, um einen Eingabeinformationsbitstrom auf orthogonalen Subträgern zur Übertragung als Ultraschallsignal durch den Körper an den ersten Knoten oder einen anderen Knoten der Vielzahl von Knoten oder an alle Knoten der Vielzahl von Knoten zu verschlüsseln; und
wobei der erste implantierbare Knoten ferner eines oder beides von einem Sensor, der zum Erfassen eines oder mehrerer biologischer Parameter betrieben wird, oder einem Aktor umfasst.

15. Verfahren zur Ultraschallübertragung durch biologisches Gewebe, Folgendes umfassend:

Bereitstellen eines Signals an einem ersten Knoten, der in einem Körper implantiert ist;
Übertragen des Signals durch biologisches Gewebe; und
Empfangen des Signals an einem zweiten Knoten;
dadurch gekennzeichnet, dass das Signal ein Informationsbitstrom ist, der auf orthogonalen Unterträgern unter Verwendung eines orthogonalen Frequenzmultiplexmodulationsverfahrens verschlüsselt ist; und dass das Signal bei Empfang entschlüsselt wird, um den Informationsbitstrom wiederzuerlangen;
und vorzugsweise ferner eines oder mehrere von Folgendem umfassend:

das Verschlüsseln des Informationsbitstroms umfasst Generieren eines basisbandmodulierten Signals als Summe über eine Anzahl von Unterträgern der zu übertragenden Symbole in Abhängigkeit einer Frequenzbeabstandung zwischen den Unterträgern für einen Zeitblock einer gegebenen Dauer;
Senden von Symbolen auf einem Satz besetzter Unterträger, die einen Teilsatz verfügbarer Unterträger umfassen, wobei die besetzten Unterträger festgelegt oder zufällig ausgewählt sind und sich in nachfolgenden Blöcken innerhalb eines Frames ändern;
Senden von Symbolen in Blöcken zu festgelegten oder zufällig ausgewählten Zeitchips innerhalb eines Zeitrahmens;
Bereitstellen einer Vorwärtsfehlerkorrektur, wobei die Vorwärtsfehlerkorrektur das Hinzufügen von Paritätssymbolen unter Verwendung eines Blockcodes oder eines Faltungscodes umfasst;
Erfassen eines eingehenden Frames vom ersten Knoten und Identifizieren als Ausgangspunkts eines Pakets am zweiten Knoten; umfassend Korrelieren des empfangenen Ultraschallsignals mit einem lokalen Exemplar einer Präambel, die jedem OFDM-Frame vorausgeht, wobei die Präambel eine Pseudogeräuschsequenz oder eine Chirp-Sequenz umfasst; und
Maximieren einer Übertragungsrate zwischen dem Sender und dem Empfänger am zweiten Knoten durch Auswählen einer Augenblicksleistung, einer Anzahl belegter Unterträger, einer Zeitsprung-Framelänge, einer Vorwärtsfehlerkorrekturcodierungsrate und einer Modulationsrate auf Grundlage eines Störungsniveaus und einer Kanalqualität, die am Empfänger gemessen werden, und eines Störungsniveaus, das durch den Empfänger in Kommunikation mit anderen Knoten generiert wird.

**Revendications**

1. Système de transmission de données par ultrasons à travers un tissu biologique comprenant :

   un réseau comprenant une pluralité de nœuds, au moins une partie des nœuds pouvant être implantés dans un corps ; et
   un premier nœud pouvant être implanté dans le corps et comprenant un transducteur à ultrasons et un émetteur, et un second nœud comprenant un récepteur à ultrasons ;
   le système étant **caractérisé en ce que** l'émetteur au niveau du premier nœud comporte un générateur de signal de multiplexage par répartition orthogonale de la fréquence (OFDM) permettant de coder un train de bits d'informations d'entrée sur des sous-porteuses orthogonales à transmettre sous forme de signal ultrasonore à travers le corps au récepteur à ultrasons au niveau du second nœud ; et
   **en ce que** le récepteur à ultrasons au niveau du second nœud permet de décoder le signal ultrasonore reçu en provenance du premier nœud pour récupérer le train de bits d'informations.

2. Système selon la revendication 1, dans lequel le générateur de signal OFDM permet de générer un signal modulé en bande de base comme une somme par rapport à un certain nombre de sous-porteuses des symboles à transmettre en fonction d'un espacement de fréquence entre les sous-porteuses pour un bloc temporel d'une durée donnée.

3. Système selon la revendication 2, dans lequel le générateur de signal OFDM permet d'introduire un temps de garde entre des blocs temporels, et de préférence dans lequel le temps de garde comprend un silence ou une répétition du bloc temporel.

4. Système selon la revendication 2, comprenant en outre une unité de mappage de symboles pour mapper le train de bits dans une constellation d'un schéma de modulation, et de préférence dans lequel le schéma de modulation comprend une modulation par déplacement de phase M ou une modulation d'amplitude en quadrature M.

5. Système selon la revendication 1, dans lequel le générateur de signal OFDM comprend :

   un convertisseur série-parallèle pour convertir le train de bits d'informations d'entrée en une pluralité de chaînes de données parallèles,
   un transformateur de Fourier inverse pour générer une représentation du domaine fréquentiel du train de bits d'informations d'entrée,
   un convertisseur parallèle-série pour convertir la représentation du domaine fréquentiel en un flux en série, et
   un convertisseur élévateur pour convertir le signal en une fréquence porteuse à transmettre à travers le tissu biologique.

6. Système selon la revendication 1, dans lequel le récepteur comprend :

   un convertisseur abaisseur pour convertir le signal reçu en un signal en bande de base,
   un convertisseur série-parallèle pour convertir le signal en bande de base en une pluralité de chaînes de données parallèles,
   un transformateur de Fourier ; et
   un convertisseur parallèle-série pour convertir les chaînes de données parallèles en une chaîne de données en série.

7. Système selon la revendication 1, dans lequel le générateur de signal OFDM permet d'envoyer des symboles sur un ensemble de sous-porteuses occupées comprenant un sous-ensemble de sous-porteuses disponibles, et de préférence un ou plusieurs parmi :

   dans lequel les sous-porteuses occupées sont fixes ou sélectionnées de manière aléatoire et changent par blocs consécutifs dans une trame ; et
   dans lequel les sous-porteuses occupées sont sélectionnées par une séquence de sauts de fréquence pseudo-aléatoire générée en amorçant un générateur de nombres aléatoires avec une identification propre à l'émetteur.

**8.** Système selon la revendication 1, dans lequel le générateur de signal OFDM permet d'envoyer des symboles en blocs à des éléments temporels fixes ou sélectionnés de manière aléatoire dans une trame temporelle ; et de préférence un ou plusieurs parmi :

dans lequel le générateur de signal OFDM permet d'envoyer des symboles selon une séquence de sauts temporels pseudo-aléatoire générée en amorçant un générateur de nombres aléatoires avec une identification propre à l'émetteur ; et

dans lequel le récepteur permet de décoder le signal ultrasonore au niveau du récepteur en amorçant un générateur aléatoire avec l'identification de l'émetteur pour générer la même séquence de sauts temporels pseudo-aléatoire.

**9.** Système selon la revendication 1, dans lequel le générateur de signal OFDM permet de fournir une correction d'erreurs sans voie de retour, et de préférence un ou plusieurs parmi :

dans lequel la correction d'erreurs sans voie de retour comprend l'ajout de $t$ symboles de parité à $k$ symboles d'information pour former un bloc de $n$ symboles ; et

dans lequel la correction d'erreurs sans voie de retour comprend l'ajout de symboles de parité à l'aide d'un code de bloc ou d'un code de convolution.

**10.** Système selon la revendication 1, dans lequel le générateur de signal OFDM permet de fournir une ou plusieurs techniques de modulation à un niveau de sous-porteuse, à un niveau de bloc ou à un niveau de trame, et de préférence dans lequel la technique de modulation est sélectionnée pour optimiser un débit de données en fonction d'un ou de plusieurs éléments parmi un certain nombre de sous-porteuses occupées, un certain nombre d'éléments temporels par trame temporelle, un taux de codage de correction d'erreurs et un taux de modulation.

**11.** Système selon la revendication 1, dans lequel le récepteur permet de détecter une trame entrante provenant de l'émetteur et d'identifier un point de départ d'un paquet ; et de préférence un ou plusieurs parmi :

dans lequel l'identification du point de départ comprend la mise en corrélation du signal ultrasonore reçu avec une copie locale d'un préambule précédant chaque trame OFDM ; et

dans lequel le préambule comprend une séquence de pseudo-bruit ou une séquence de chirp.

**12.** Système selon la revendication 1, comprenant en outre un ou plusieurs parmi :

dans lequel le récepteur permet de déterminer un rapport signal sur interférence plus bruit en fonction d'une puissance instantanée, d'une longueur de trame de saut temporel et d'un certain nombre de sous-porteuses occupées ;

dans lequel le récepteur permet de maximiser un taux de transmission entre l'émetteur et le récepteur en sélectionnant une puissance instantanée, un certain nombre de sous-porteuses occupées, une longueur de trame de saut temporel, un taux de codage de correction d'erreurs sans voie de retour et un taux de modulation sur la base d'un niveau d'interférence et de qualité de canal mesuré au niveau du récepteur et d'un niveau d'interférence généré par le récepteur en communication avec d'autres nœuds ;

dans lequel le récepteur permet de déterminer une valeur de puissance instantanée, un certain nombre de sous-porteuses occupées, une longueur de trame de saut temporel, un taux de codage de correction d'erreurs sans voie de retour et un taux de modulation qui maximisent un débit de données ;

dans lequel le récepteur permet en outre de maximiser le débit de données soumis à un rapport signal sur interférence plus bruit par nœud supérieur à une valeur minimale et à un débit de données par nœud supérieur à une valeur minimale ;

dans lequel le récepteur permet en outre de déterminer un taux d'énergie, le taux d'énergie comprenant une énergie par bit ou une puissance moyenne rayonnée par seconde ; et

dans lequel le récepteur permet en outre de minimiser le taux d'énergie soumis à un rapport signal sur interférence plus bruit par nœud supérieur à une valeur minimale et à un débit de données par nœud supérieur à une valeur minimale.

**13.** Système selon la revendication 1, dans lequel l'émetteur permet d'ouvrir une communication avec un récepteur sur un canal de contrôle commun à l'aide d'une procédure d'établissement de liaison bidirectionnelle, et l'émetteur permet de transmettre sur un canal dédié au récepteur une séquence de sauts de fréquence et une séquence de sauts temporels après avoir reçu un signal prêt à transmettre provenant du récepteur, et le récepteur permet de transmettre à l'émetteur une stratégie de transmission optimale ; et de préférence un ou plusieurs parmi :

dans lequel l'émetteur permet de déterminer la séquence de sauts de fréquence et la séquence de sauts temporels en amorçant un générateur de nombres aléatoires avec une identification propre à l'émetteur ;
dans lequel la stratégie de transmission optimale comprend un certain nombre de sous-porteuses occupées, une longueur de trame de saut temporel, un taux de codage de correction d'erreurs sans voie de retour et un taux de modulation ; et
dans lequel le récepteur permet d'échanger des informations concernant un niveau d'interférence tolérable sur le canal de contrôle commun avec d'autres nœuds de réception.

14. Système selon la revendication 1, dans lequel le premier nœud comprend en outre un récepteur à ultrasons pour décoder un signal ultrasonore reçu en provenance d'un autre nœud parmi la pluralité de nœuds ; et de préférence un ou plusieurs parmi :

dans lequel le second nœud comprend en outre un transducteur à ultrasons et un émetteur, l'émetteur au niveau du second nœud comportant un générateur de signal de multiplexage par répartition orthogonale de la fréquence (OFDM) permettant de coder un train de bits d'informations d'entrée sur des sous-porteuses orthogonales à transmettre sous forme de signal ultrasonore à travers le corps au premier nœud ou à un autre nœud parmi la pluralité de nœuds ou à tous les nœuds de la pluralité de nœuds ; et
dans lequel le premier nœud pouvant être implanté comprend en outre l'un ou les deux parmi un capteur permettant de détecter un ou plusieurs paramètres biologiques ou un actionneur.

15. Procédé de transmission de données par ultrasons à travers un tissu biologique comprenant :

au niveau d'un premier nœud implanté dans un corps, la fourniture d'un signal ;
la transmission du signal à travers un tissu biologique ; et
au niveau d'un second nœud, la réception du signal ;
**caractérisé en ce que** le signal est un train de bits d'informations codé sur des sous-porteuses orthogonales à l'aide d'un schéma de modulation de multiplexage par répartition orthogonale de la fréquence ; et **en ce que** le signal est décodé à la réception pour récupérer le train de bits d'informations ;
et de préférence comprenant en outre un ou plusieurs parmi :

le codage du train de bits d'informations comprend la génération d'un signal modulé en bande de base comme une somme par rapport à un certain nombre de sous-porteuses des symboles à transmettre en fonction d'un espacement de fréquence entre les sous-porteuses pour un bloc temporel d'une durée donnée ;
l'envoi de symboles sur un ensemble de sous-porteuses occupées comprenant un sous-ensemble de sous-porteuses disponibles, dans lequel les sous-porteuses occupées sont fixes ou sélectionnées de manière aléatoire et changent par blocs consécutifs dans une trame ;
l'envoi de symboles en blocs à des éléments temporels fixes ou sélectionnés de manière aléatoire dans une trame temporelle ;
la fourniture d'une correction d'erreurs sans voie de retour, dans lequel la correction d'erreurs sans voie de retour comprend l'ajout de symboles de parité à l'aide d'un code de bloc ou d'un code de convolution ;
au niveau du second nœud, la détection d'une trame entrante provenant du premier nœud et l'identification d'un point de départ d'un paquet, comprenant la mise en corrélation du signal ultrasonore reçu avec une copie locale d'un préambule précédant une trame OFDM, dans lequel le préambule comprend une séquence de pseudo-bruit ou une séquence de chirp ; et
au niveau du second nœud, la maximisation d'un taux de transmission entre l'émetteur et le récepteur en sélectionnant une puissance instantanée, un certain nombre de sous-porteuses occupées, une longueur de trame de saut temporel, un taux de codage de correction d'erreurs sans voie de retour et un taux de modulation sur la base d'un niveau d'interférence et de qualité de canal mesuré au niveau du récepteur et d'un niveau d'interférence généré par le second nœud en communication avec d'autres nœuds.

*FIG. 1*

*FIG. 2*

**FIG. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013033966 A
- US 2014016558 A
- EP 1115221 A
- US 2010080265 A
- US 2014269396 A
- US 2007238482 A
- US 2012134423 A